# EUROPEAN PATENT APPLICATION

(11) **EP 2 641 631 A1**
(43) Date of publication of application: **25.09.2013**
(21) Application number: 11842206.2
(22) Date of filing: 17.11.2011
(51) Int. Cl.: A61M 16/10

(54) **OVERHEATING DETECTION UNIT AND OXYGEN-CONCENTRATING DEVICE**

(30) Priority: 18.11.2010 JP 2010258268
(71) Applicant: Ikiken Co., Ltd., Saitama 350-1331 (JP)
(72) Inventor: ENOMOTO, Hisashi, Sayama-shi Saitama 350-1331 (JP); SUGAWARA, Kimio, Sayama-shi Saitama 350-1331 (JP)
(74) Representative: Kremer, Simon Mark
(86) International application number: PCT/JP2011/006413
(87) International publication number: WO 2012/066784

(57) **Abstract**

Provided are an overheating detection unit and an oxygen concentrator capable of securing safety since, by attaching the overheating detection unit between the oxygen concentrator and a cannula, it is possible to reliably detect a high-temperature environment to cut the supply of oxygen when a user inhales oxygen using the cannula and is exposed to a fire or an abnormal high-temperature environment. An overheating detection unit (300) includes a main body (302) including a connecting member (330) connected to an oxygen outlet (15) and an oxygen outlet portion (310) connected to a tube (23) and also includes a housing (301) that covers the main body (302). The main body (302) includes a temperature sensor (453) disposed in the oxygen outlet portion (310) so as to detect the temperature of the oxygen outlet portion and a blocking structure portion (402) that blocks a passage between the connecting member (330) and the oxygen outlet portion (310) to thereby cut the supply of oxygen when the temperature sensor detects a temperature that is equal to or higher than a predetermined temperature.

## Description

### Technical Field

The present invention relates to an overheating detection unit and an oxygen concentrator capable of preventing spreading of fire to the oxygen concentrator, fire of the oxygen concentrator itself, and the like in the case of exposure to an abnormal high-temperature environment such as fire by attaching the overheating detection unit between the oxygen concentrator and a cannula.

### Background Art

As an example of an oxygen concentrator, an adsorption-type oxygen concentrator that compresses input raw material air to generate compressed air and supplies the compressed air to an adsorption cylinder that stores an adsorbent so that nitrogen is adsorbed to the adsorbent and oxygen is generated and a membrane separation-type oxygen concentrator that uses an oxygen selective permeating membrane which is a polymer membrane of which the oxygen permeation coefficient is larger than the nitrogen permeation coefficient are known. In the adsorption-type oxygen concentrator, a zeolite is mainly used as an example of the adsorbent that adsorbs nitrogen. Moreover, in the oxygen selective permeating membrane-type oxygen concentrator, polydimethylsiloxane-polycarbonate copolymer, poly(4-methylpentene-1), a polyphenylene oxide, a porphyrin complex containing film, and the like are used, for example. Moreover, oxygen concentrated to 90% or higher is stored in a tank, and a state where the oxygen can be supplied at a predetermined flow rate from the tank with the aid of a pressure-reducing valve and a flow rate setting device is created. In this manner, the user can inhale oxygen using a tool such as a nasal cannula. When the oxygen concentrator is installed at a place where an AC power supply (utility AC power supply) can be used, for example, a domiciliary oxygen therapy patient having a deteriorated lung function can safely inhale oxygen even while sleeping to have a good sleep.

Moreover, the oxygen concentrator used for a longterm oxygen inhalation therapy which is effective as a therapeutic method for a patient who suffers from respiratory disease such as chronic bronchitis is generally not transportable and is not configured for the patient to go outside while carry the same.
Moreover, an oxygen concentrator in which a temperature sensor is provided in a nasal cannula, and generation of concentrated oxygen is stopped when the temperature reaches 50°C is also proposed (see Patent Literature 1).

### Citation List

### Patent Literature

Patent Literature 1: Japanese Patent Application Laid-open No. 2009-183544

### Summary of Invention

### Technical Problem

The oxygen concentrator disclosed in Patent Literature 1 has a configuration in which a temperature sensor is disposed in the halfway of the nasal cannula, and the supply of oxygen is stopped based on the signal detected by the temperature sensor. However, in such a very simple structure disclosed in Patent Literature 1 that the supply of oxygen is stopped at approximately 50°C, there is a possibility that the supply of oxygen is cut just because a heating appliance is located in the room where the oxygen concentrator is used, and the heat radiated from the heating appliance reaches the oxygen concentrator. There is another possibility that the oxygen concentrator may not operate in a closed room in the summer just because the temperature of the environment where the oxygen concentrator is placed increases. Thus, the oxygen concentrator is inconvenient to use and is not useful.

Therefore, an object of the present invention is to provide an overheating detection unit and an oxygen concentrator capable of securing safety since, by attaching the overheating detection unit between the oxygen concentrator and a cannula, it is possible to reliably detect a high-temperature environment to cut the supply of oxygen when a user inhales oxygen using the cannula and is exposed to a fire or an abnormal high-temperature environment.

### Solution to Problem

An overheating detection unit according to the present invention is an overheating detection unit disposed between an oxygen outlet of an oxygen concentrator that generates concentrated oxygen from raw material air and that has the oxygen outlet outputting the concentrated oxygen and a nasal cannula having a coupler socket, or between the oxygen outlet and the coupler socket provided at one end of a tube that is connected to the nasal cannula, so as to detect an overheating state, the overheating detection unit comprising: a main body including a connecting member connected to the oxygen outlet and also including an oxygen outlet portion connected to the coupler socket; and a housing covering the main body, the main body including: a temperature sensor disposed in the oxygen outlet portion so as to detect a temperature of the oxygen outlet portion; and a blocking structure portion blocking a passage between the connecting member and the oxygen outlet portion so as to cut supply of oxygen when the temperature sensor detects a temperature that is equal to or higher than a predetermined temperature.
According to the above configuration, it is possible to secure safety since by attaching the overheating detection unit between the oxygen concentrator and the cannula, it is possible to reliably detect a high-temperature environment to cut the supply of oxygen when the user is inhaling oxygen using the cannula and is exposed to a fire or an abnormal high-temperature environment.

Preferably, the housing includes a first cover that is fixed to the main body, a second cover that is fixed to the main body and detachably attached to the first cover, and a protection cover that is fixed to the main body so as to cover bottom portions of the first and second covers, and a first opening for exposing the oxygen outlet portion is formed in the first and second covers, and a second opening for exposing an end of the connecting member is formed in a bottom portion of the protection cover.
According to the above configuration, the main body can be protected by being covered by the housing and the protection cover. The oxygen outlet portion is exposed from the housing and thus can be easily connected to the tube. The connecting member is exposed from the protection cover and thus can be easily connected to the oxygen outlet of the oxygen concentrator.

Preferably, the protection cover is detachably fixed to the main body by one screw.
According to the above configuration, by using only one screw, the protection cover can be reliably fixed to the main body, and the operation of attaching and removing the protection cover can be facilitated.
Preferably, the protection cover has a convex fitting portion that is fitted to a step portion formed in the oxygen concentrator in a state where the oxygen outlet is connected to the connecting member.
According to the above configuration, the overheating detection unit can be held by being reliably fitted to the oxygen concentrator in a state where the connecting member of the overheating detection unit is connected to the oxygen outlet of the oxygen concentrator.

Preferably, the protection cover is formed of a transparent resin, and the protection cover has a lens portion for guiding light of an oxygen lamp provided in the oxygen concentrator so as to emit light to display that oxygen is output from the oxygen outlet to a periphery of the overheating detection unit.
According to the above configuration, even when the overheating detection unit is covered on the oxygen lamp, the user can easily observe the light of the oxygen lamp through the lens portion.
Preferably, the lens portion guides the light of the oxygen lamp of the oxygen concentrator to a plurality of positions of an outer circumference of the second cover.
According to the above configuration, the user can easily observe the light of the oxygen lamp at the plurality of positions of the outer circumference of the second cover.

Preferably, the blocking structure portion includes a blocking member that presses an elastically deformable tube that connects the connecting member and the oxygen outlet portion to thereby cut the supply of oxygen.
According to the above configuration, it is possible to easily and reliably cut the supply of oxygen just by pressing the elastically deformable tube that connects the connecting member and the oxygen outlet portion.
Preferably, the overheating detection unit further includes a display that is provided on the housing so as to inform by changing emission colors a user of a normal operation state, a cut-off state of the oxygen when overheating is detected, and a battery replacement state.
According to the above configuration, the user can reliably observe a plurality of states of the overheating detection unit based on different emission colors.

An oxygen concentrator according to the present invention includes a compressed air generator that compresses raw material air to generate compressed air; an oxygen outlet that outputs oxygen obtained from the compressed air; and the overheating detection unit described above.
According to the above configuration, by attaching the overheating detection unit between the oxygen concentrator and the cannula, it is possible to reliably detect a high-temperature environment to secure safety when the user is inhaling oxygen using the cannula and is exposed to a fire or an abnormal high-temperature environment.

### Advantageous Effects of Invention

According to the present invention, it is possible to provide an overheating detection unit and an oxygen concentrator capable of securing safety since by attaching the overheating detection unit between the oxygen concentrator and the cannula, it is possible to reliably detect a high-temperature environment to cut the supply of oxygen and to secure safety when the user is inhaling oxygen using the cannula and is exposed to a fire or an abnormal high-temperature environment.

### Brief Description of Drawings

FIG. 1 is an external perspective view showing a preferred embodiment of an oxygen concentrator according to the present invention.
FIG. 2 is a plan view showing an operation panel of the oxygen concentrator shown in FIG. 1.
FIG. 3 is a perspective exploded view as seen from the back side in order to show an inner configuration of the oxygen concentrator.
FIG. 4 is a system diagram of the oxygen concentrator.
FIG. 5 is a perspective view showing the operation panel of the oxygen concentrator and an overheating detection unit.
FIG. 6(A) is a perspective view showing the operation panel, and FIG. 6(B) is a plan view showing the operation panel.
FIG. 7 is a perspective view showing the overheating detection unit.
FIG. 8 is a cross-sectional view along line C-C, of the overheating detection unit and the operation panel shown in FIG. 6(B).
FIG. 9 is a perspective view showing a main body.
FIG. 10 is a view showing the overheating detection unit.
FIG. 11 is a perspective view showing the structure of a protection cover and a battery replacement structure.
FIG. 12 is a view showing a main body including a tube blocking structure portion.
FIG. 13 is a view showing a state where the tube of the main body is not pressed by a cam but is open.
FIG. 14 is a view showing a state where the tube of the main body is pressed and blocked by the cam.
FIG. 15 is a view showing an electric contact of the battery and a setting position of a thermistor.
FIG. 16 is a diagram showing an electric circuit.

### Description of Embodiments

Hereinafter, preferred embodiments of the present invention will be described in detail with reference to the drawings.
The various limitations that are technically preferable are added to the embodiments described below because the embodiments are preferred specific examples of the present invention. However, the scope of the present invention is not limited to these embodiments unless there is an explicit description for limiting the scope of the present invention.

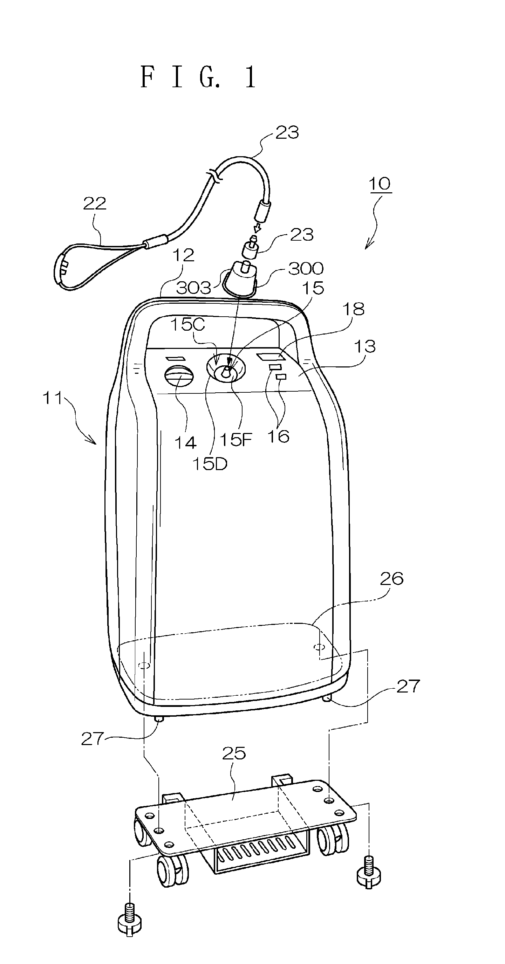

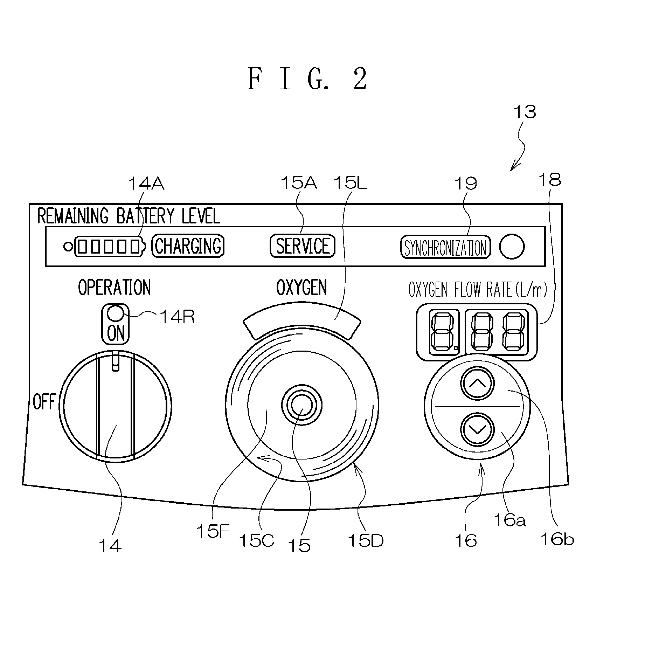

FIG. 1 is an external perspective view showing a preferred embodiment of an oxygen concentrator according to the present invention, and FIG. 2 is a plan view showing an operation panel of the oxygen concentrator shown in FIG. 1.
An oxygen concentrator 10 shown in FIG. 1 is an example of a pressure swing adsorption-type oxygen concentrator and includes a vertically long body case 11 having a handle 12 serving as a lug at an upper end thereof, for example. An operation panel 13 is provided near the upper end of the body case 11 so as to be slightly inclined frontward. A dial-type power switch 14, an oxygen outlet 15, an oxygen flow rate setting switch 16, and an oxygen flow rate display portion 18 that displays segment numbers using an LED or a liquid crystal display, for example are disposed in the operation panel 13 in that order from the left.

As shown in FIGS. 1 and 2, a step portion 15D is formed at a central position of the operation panel 13. This step portion 15D is a circular depression, and the step portion 15D includes a flat circular bottom portion 15F and an inner circumferential portion 15C. A diameter on the front surface of the step portion 15D is larger than the diameter on the bottom portion 15F. The oxygen outlet 15 is provided at the central position of the bottom portion 15F of the step portion 15D so as to protrude vertically to the bottom portion 15F. The oxygen outlet 15 is formed of a metal material that has high heat conductivity and rarely becomes rusty, for example, and is preferably formed of a copper alloy, an aluminum alloy, or the like.
Since the step portion 15D is formed in the operation panel 13 in this manner, the oxygen outlet 15 can be positioned in the step portion 15D, and a portion of an overheating detection unit 300 shown in FIG. 1 can be accurately aligned and fitted to be detachably attached to the step portion 15D as compared to a case where the oxygen outlet 15 is provided so as to protrude from the surface of the operation panel 13. The overheating detection unit 300 can be also called an overheating detection adapter.

The overheating detection unit 300 shown in FIG. 1 is mounted by being detachably connected between the oxygen outlet 15 of the oxygen concentrator 10 and a nasal cannula 22 having a coupler socket 23 that forms a connecting portion at one end thereof. The overheating detection unit 300 is used for reliably detecting a high-temperature environment to secure safety when a user is exposed to fire or an abnormal high-temperature environment when the user is inhaling oxygen using the nasal cannula 22.

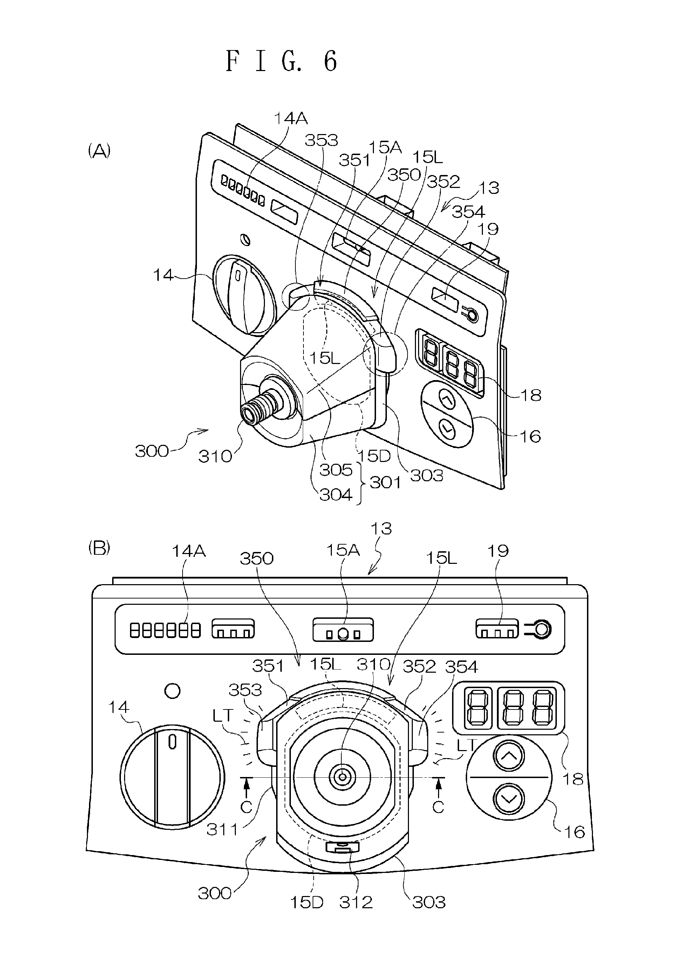

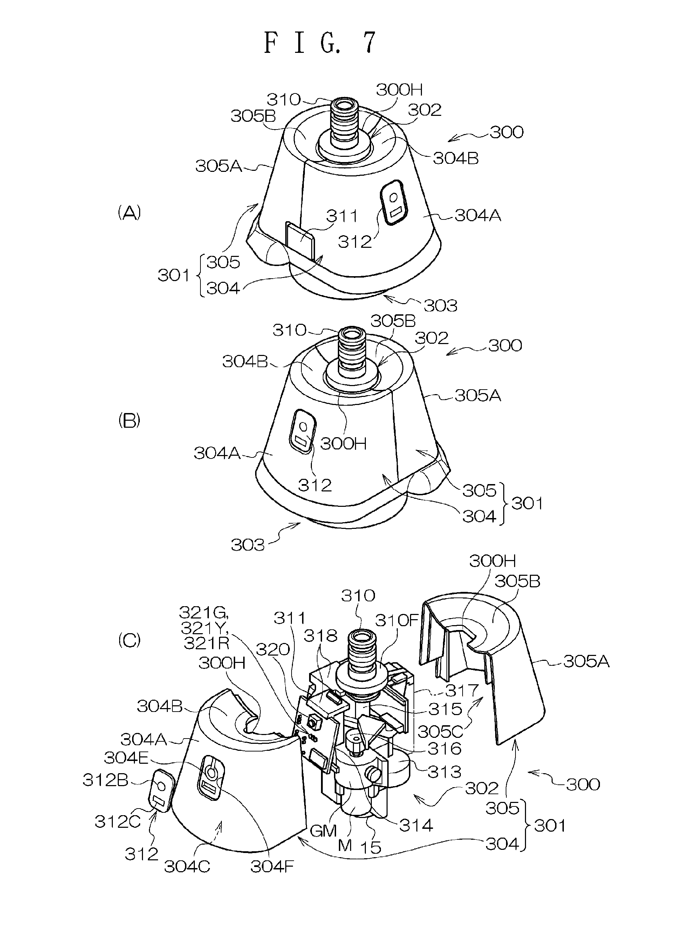

In FIG. 1, the overheating detection unit 300 is shown to be separated above the oxygen outlet 15. A protection cover 303 (see FIGS. 6 and 7 and other figures) which is a portion of the overheating detection unit 300 is detachably mounted by being fitted into the step portion 15D so as to make close contact with the inner circumferential portion 15C and the bottom portion 15F of the step portion 15D. In a state where the protection cover 303 is fixed by being fitted into the step portion 15D in this manner, the overheating detection unit 300 can connect to the oxygen outlet 15 and the coupler socket 23 so that the oxygen outlet 15 of the oxygen concentrator 10 communicates with the opening of the coupler socket 23 of the nasal cannula 22, which will be described later.

Four rubber legs 27 are fixed to four corners of a bottom cover 26 of the body case 11 shown in FIG. 1 so as to prevent sideslip when the oxygen concentrator is used in a state of being installed on a floor. A carrier 25 used during movement such as when one goes out can be fixed to the bottom cover 26 by two fixing screws. Holes in which the rubber legs 27 are accommodated are formed at the corresponding positions of the carrier 25, and freely movable casters formed of a resin are disposed at four corners.

FIG. 2 shows the operation panel 13 shown in FIG. 1 at an enlarged scale.
The power switch 14 shown in FIG. 2 is operated between an OFF position shown in the figure and an ON position where the switch is rotated clockwise by approximately 90°. An operation state lamp 14R in which, for example, a light emitting LED or the like that is lit in green and red is provided at the position corresponding to the ON position of the power switch 14. Moreover, a battery level monitor 14A is provided above the operation state lamp.
An alarm display portion 15A in which the character "Service" or a character display or the like corresponding to this is printed crosswise is disposed above the oxygen outlet 15 at the center. An oxygen lamp 15L in which, for example, a light emitting LED that is lit in green, red and yellow is provided below the alarm display portion 15A. The oxygen lamp 15L is one that displays by lighting that oxygen comes out from the oxygen outlet 15, and in the example of FIG. 2, the oxygen lamp 15L is formed approximately linearly along the step portion 15D.

The oxygen flow rate setting switch 16 shown in FIG. 2 is provided as flat switches 16a and 16b in which UP and DOWN arrows are printed. The oxygen flow rate setting switch 16 is configured to set an oxygen flow rate when pressurizing oxygen concentrated to approximately 90% or higher, for example, from 0.25 liters (L) per minute to 5 L per minute at the maximum with a step of 0.25 L or 0.5 L. The oxygen flow rate setting switch 16 can change the oxygen generation ability by displaying the flow rate setting value at that time on the oxygen flow rate display portion 18 above the oxygen flow rate setting switch 16. A synchronization lamp 19 is provided in order to inform the user, by lighting or blinking, of the fact that the oxygen concentrator is operating in an interrupted supply state where concentrated oxygen is supplied in synchronization with breathing.

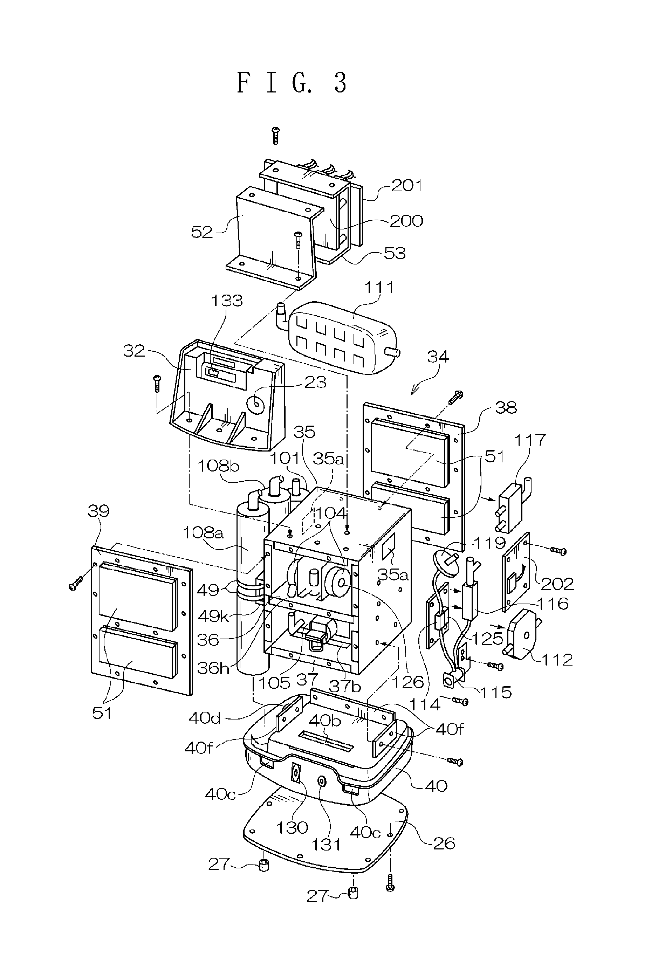

FIG. 3 is a perspective exploded view as seen from the back side in order to show an inner configuration of the oxygen concentrator 10. As shown in FIG. 3, the rubber legs 27 are fixed from the lower side to four corners of the bottom cover 26 formed of a resin. The bottom cover 26 is fixed to a bottom surface of a base member 40 formed of a resin using a plurality of fixing screws.
The base member 40 has such a box shape that walls continuously formed downward from four surfaces are integrally molded, and connectors 131 and 130 are fixed to the wall of a rear surface. Exhaust ports 40c that face exhaust ports of a rear cover (not shown) provided in the body case 11 shown in FIG. 1 and communicate with an internal power chamber are formed as shown in FIG. 3, and final external ventilation is performed by these exhaust ports 40c. The upper surface of the base member 40 is formed to be flat as shown in the figure, and standing portions 40f in which holes are formed so as to fix the base member 40 to a 2-stage sound-proof chamber 34 from the three sides of the left, right, and rear surfaces of the 2-stage sound-proof chamber 34 are molded integrally with the upper surface of the base member 40. Moreover, an exhaust opening 40b that communicates with the power chamber is further formed on the upper surface of the base member 40.

The 2-stage sound-proof chamber 34 shown in FIG. 3 is formed of a light metal board as a closed box 35 in which two blast fans 104 are fixed to an upper-stage member 36 that is configured to be taken in and out from the lateral side at the front of the figure, and a compressor 105 as a compressed air generator provided on a lower-stage member 37 that is configured to be taken in and out from the same lateral side is arranged in a vibration-proof state.
In the 2-stage sound-proof chamber 34 has a sound-proof chamber cover 39 shown at the front side as shown in the figure and a sound-proof chamber cover 38 shown at the rear side are fixed by a plurality of fixing screws. A soundproofing material 51 is laid inside the 2-stage soundproof chamber 34. Moreover, a sheet-shaped damping member formed of a raw material which is a mixture of synthetic rubber and a special resin material is laid on the outer circumferential surface, and thus, the 2-stage soundproof chamber 34 itself which is a thin aluminum board does not vibrate with resonance or the like.

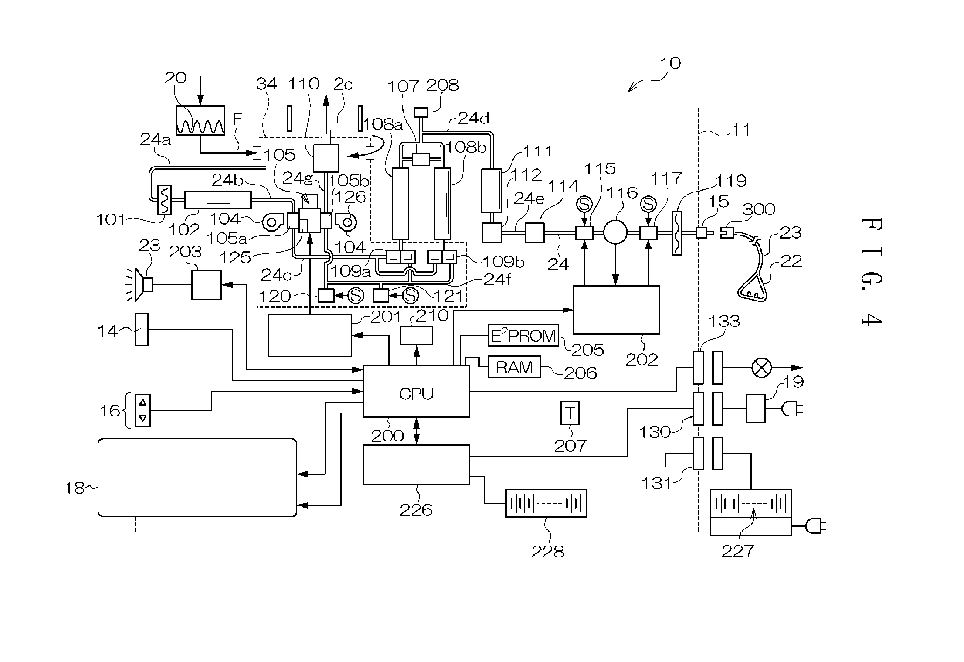

A first opening 35a (depicted by solid and broken lines) is formed on an upper portion of each of the left and right side walls of the upper-stage member 36 of the 2-stage soundproof chamber 34 shown in FIG. 3 so as to introduce outside air into the 2-stage soundproof chamber 34. A plurality of fixing holes 36h for fixing a pipe 24 described in FIG. 4 using a rubber bush is formed on the upper-stage member 36 and is configured to support the pipe 24 and perform a vibration-proofing function in collaboration with the rubber bush.
Moreover, an inverter-controlled sirocco fan, for example, can be used as the blast fans 104. The blast fans 104 are fixed to the upper-stage member 36 using brackets so that respective blast ports face downward. Three-way switching valves 109a and 109b and the like shown in FIG. 4 are disposed between the blast fans 104. A fan rotation detector 126 is provided in each blast fan 104.
Cylindrical adsorption cylinders 108a and 108b are disposed on the left side wall of the 2-stage soundproof chamber 34 shown in FIG. 3 so as to be in parallel to an intake buffer tank 101. The adsorption cylinders 108a and 108b pass through a band 49 and are then fixed to a fixing jig 49k that is fixed to the side wall by squeezing the band 49. In this case, although the adsorption cylinders 108a and 108b are mounted on the upper surface of the base member 40, a portion of the buffer tank 101 having a large entire length is fixed by being inserted into an opening 40d.

A product tank 111 shown in FIG. 3 is formed of a blow-molded polypropylene resin and is disposed above the 2-stage soundproof chamber by being laid in a longitudinal direction as shown in the figure. A shield plate 32 is also formed of a resin in order to decrease a weight and is provided with a speaker 23 and an external connector 133. A reinforcing attachment portion that is fixed to an upper outer wall of the 2-stage soundproof chamber 34 using a fixing screw is integrally molded to the shield plate 32. Heat radiating members 52 and 53 are fixed to the upper wall of the 2-stage soundproof chamber 34 using fixing screws, and a central control unit 200 (a substrate including a CPU described later) that has the functions as determination means for making various determinations in an entire operation process of the oxygen concentrator 10 and a control unit that controls the entire operation process and a substrate 201 that includes a motor control unit are fixed in a standing state so as to enhance a heat radiating effect. An oxygen sensor 114, a proportional aperture valve 115, a pressure regulator 112, a flow rate sensor 116, a demand valve 117, a circuit board 202, and a temperature sensor 125 are fixed to the right side wall of the 2-stage soundproof chamber 34.

FIG. 4 is a system diagram (piping diagram) of the oxygen concentrator 10.
In FIG. 4, double lines are flow paths of air and oxygen and nitrogen gases and generally indicate pipes 24a to 24g. Moreover, narrow solid lines indicate power supply or electrical signal wires.
In the following description, a case where one in which compressing means (compressed air generator) and decompressing means (negative pressure generator) are integrated is used as the compressor 105 will be described. However, it goes without saying that the present invention is not limited to this, and the compressed air generator and the negative pressure generator may be separated from each other. A front cover and a rear cover (a portion of the body case 11) that introduce outside air through an intake port and discharge the same to the outside through an exhaust port 2c are depicted by broken lines in FIG. 4 as a closed container.

In FIG. 4, description will be made sequentially along the flow of introduced air.
Air (outside air) passes through an outside air introduction filter 20 included in a filter replacement cover and is introduced into the oxygen concentrator 10 in the direction indicated by arrow F. This air enters into the 2-stage soundproof chamber 34 by being blown by the pair of blast fans 104. As described above, in the 2-stage soundproof chamber 34, air enters into the 2-stage soundproof chamber 34 through openings that are formed on the side surfaces of the 2-stage soundproof chamber 34 (depicted by broken lines) in which the blast fans 104 are arranged on the upper-stage member and the compressor 105 are arranged on the lower-stage member in a vibration-proof state. In order to supply a portion of the air as raw material air to compressing means 105a of the compressor 105, an opening of the pipe 24a is provided inside the 2-stage soundproof chamber 34, and an intake filter 101 that performs secondary filtering and a large-capacity intake muffler 102 are provided halfway the pipe 24a. With this configuration, the intake sound of the raw material air stays in the 2-stage soundproof chamber 34 so that the intake sound is reduced.

In the 2-stage soundproof chamber 34 shown in FIG. 4, the compressor 105 in which compressing means 105a that compresses the raw material air to generate compressed air and decompressing means 105b are preferably integrated is fixed in a vibration-proof state. The temperature sensor 125 is disposed near the compressor 105 at a position where a temperature environment is substantially the same as the compressor 105 (see FIG. 3).
Subsequently, the filtered raw material air is pressurized by the compressing means 105a of the compressor 105 to become compressed air. In this case, since the compressed air is delivered to the pipe 24c in a heated state, the pipe 24c may be a light metal pipe having an excellent heat radiating effect and be cooled by the wind blown from the blast fan 104. By cooling the compressed air in this manner, oxygen can be sufficiently concentrated to approximately 90% using a zeolite which is an adsorbent of which the function deteriorates at a high temperature as an adsorbent for generating oxygen by adsorbing nitrogen.

The compressed air is alternately supplied to the first and second adsorption cylinders 108a and 108b serving as adsorbing portions through the pipe 24c. Thus, switching valves (three-way switching valves) 109a and 109b are connected as shown in the figure. In order to desorb unnecessary gas of the switching valves 109a and 109b and the first and second adsorption cylinders 108a and 108b (in order to perform purging (purification)), a plurality of (at least two) first and second negative pressure destruction valves (pressure regulating valves) 120 and 121 is disposed in series in the pipe 24f that communicates with the decompressing means 105b. By opening these first and second negative pressure destruction valves (pressure regulating valves) 120 and 121 to control the pressure inside the pipe 24f up to an atmospheric pressure during a pressure equalizing process when the flow rate is a predetermined value or smaller, vibration of and power consumed by the compressor are suppressed.

A zeolite is used as an example of a catalyst adsorbent stored in the first and second adsorption cylinders 108a and 108b shown in FIG. 4.
On the other hand, a check valve and a pressure equalizing valve 107 that includes a throttle valve and an on-off valve are connected to branch off from the outlet on the upper side of the first and second adsorption cylinders 108a and 108b. Moreover, the downstream side of the pressure equalizing valve 107 converges to the pipe 24d which is connected to the product tank 111 that serves as a container for storing the separated and generated oxygen having a concentration of approximately 90% or more. Moreover, a pressure sensor 208 that detects the pressure inside the first and second adsorption cylinders 108a and 108b is connected as shown in the figure.

A pressure regulator 112 that automatically regulates the oxygen pressure on the outlet side to be constant is connected to the pipe 24e on the downstream side of the product tank 111 shown in FIG. 4. The zirconium oxide-type or ultrasound-type oxygen (concentration) sensor 114 is connected to the downstream side of the pressure regulator 112 so as to detect the oxygen concentration intermittently (every 10 to 30 minutes) or continuously. The proportional aperture valve 115 that is opened or closed in synchronization with the oxygen flow rate setting switch 16 is connected to the downstream side, and the oxygen flow rate sensor 116 is connected to a further downstream side. The demand valve 117 is connected to the downstream side of the sensor 116 via a negative pressure circuit board for breath synchronization control, and is further connected to the oxygen outlet 15 of the oxygen concentrator 10 via a sterilization filter 119.
With the above configuration, the user can inhale oxygen concentrated to approximately 90% or more with a maximum flow rate of 5 L per minute through the overheating detection unit 300, the nasal cannula 22, and the like.

Subsequently, the power system shown in FIG. 4 will be described.
The AC power connector 130 shown in FIG. 4 is connected to a switching regulator-type AC unit 19 that rectifies alternating-current (AC) power to a predetermined DC voltage. The power system includes an internal battery 228 included in the main body, an external battery 227 that is detachably attached via the connector 131, and a power control circuit 226.
The internal battery 228 and the external battery 227 are secondary batteries that can be repeatedly charged, and the internal battery 228 is charged with the power supplied from the power control circuit 226. At least the internal battery 228 used is one that can be repeatedly charged and discharged at least approximately 500 times (approximately several hundred times) and that has a management function of managing a battery level, a charge/discharge cycle count, deterioration degree, an output voltage, and the like. Preferably, the internal battery 228 has a management function capable of checking a battery level, a remaining charged capacity, and a charge/discharge count on an external mobile terminal or the like.

The external battery 227 can be charged with the power supplied from the power control circuit 226 in the state of being connected via the connector 131, and is generally repeatedly charged using a battery charger that is prepared separately. Alternatively, the external battery 227 in which an exclusively designed battery charger is integrated may be prepared.
In the configuration of the power system, the oxygen concentrator 10 is used by being automatically switched to one of three power supply states, which are a first power supply state where the oxygen concentrator 10 operates with the power supplied from the AC unit 19, a second power supply state where the oxygen concentrator 10 operates with the power supplied from the internal battery 228, and a third power supply state where the oxygen concentrator 10 operates with the power supplied from the external battery 227.
The priority sequence for this automatic switching is implemented based on control of the power control circuit 226 by the central control unit 200 so that the sequence is automatically determined in the order of the first power supply state, the third power supply state, and the second power supply state.

The AC unit 19 may be a switching regulator-type AC unit that can generate a predetermined DC voltage without being affected by a frequency difference and a voltage variation and that can be configured in a small and light configuration and may be a general transformer-type AC unit.
Moreover, the central control unit 200 of the oxygen concentrator 100 has a function of switching to an optimal operation mode according to the amount of generated oxygen. The central control unit 200 preserves the internal battery 228 in particular by performing control such that the compressor 105 and the blast fan 104 automatically rotate at a high speed when a large amount of oxygen is generated and automatically rotate at a low speed when a small amount of oxygen is generated. As a result, even when the user forgets to charge the external battery 227, it is possible to deal with an unexpected going-out, a power failure, and the like.

The motor control unit 201 that controls driving of the DC motor of the compressor 105 and the motor of the blast fan 104 and a sound control unit 203 that outputs an audio content by being connected to the speaker 23 are connected to the central control unit 200.
A ROM that stores a predetermined operation program is included in the central control unit 200, and a storage device 210, a volatile memory 205, a temporary storage device 206, and a real-time clock 207 are further connected to the central control unit 200. The central control unit 200 controls the entire oxygen concentrator 100 and can access the stored contents by connecting to a communication line or the like via the external connector 133.

The flow rate control unit 202 is connected to the central control unit 200. The flow rate control unit 202 controls the driving of the three-way switching valves 109a and 109b, the pressure equalizing valve 107, the negative pressure generator 105b for desorbing the unnecessary gas in the first and second adsorption cylinders 108a and 108b, the first and second negative pressure destruction valves 120 and 121 for controlling the pressure inside the pipe 24f, the oxygen concentration sensor 114, the proportional aperture valve 115, the flow rate sensor 116, and the demand valve 117.

By providing a variable speed controller which is variable speed control means to the motor control unit 201, it is possible to freely change the speed of the compressor 105 based on a user activity level and an environmental condition. As a result, when the demand valve 117 determines based on breath synchronization that a user's oxygen demand is relatively low, for example, because the user is sitting or sleeping, it is possible to automatically decrease the rotation speed of the compressor 105. Moreover, when it is determined that the user's oxygen demand is relatively high and the amount of oxygen demand has increased, for example, because the user is standing up or active and is in a highland, it is possible to automatically increase the rotation speed.

With the above motor control, the power consumed by the entire oxygen concentrator 10 is reduced, and the life span when the oxygen concentrator 10 is driven by a chargeable battery can be extended. Moreover, by decreasing the weight and size of the chargeable battery and decreasing the wear level of the compressor 105 to extend the life span of the compressor 105, it is possible to improve reliability.
The compressor 105 is one that has the functions of generating both compressed air and negative pressure and the rotation speed is automatically controlled according to the flow rate of the output oxygen.

Here, a fan motor that drives the blast fan 104 which is a cooling fan is controlled so as to rotate at a desired rotation speed by controlling voltages according to pulse-width modulation (PWM), for example. In this manner, it is possible to easily control the rotation speed of the fan motor.

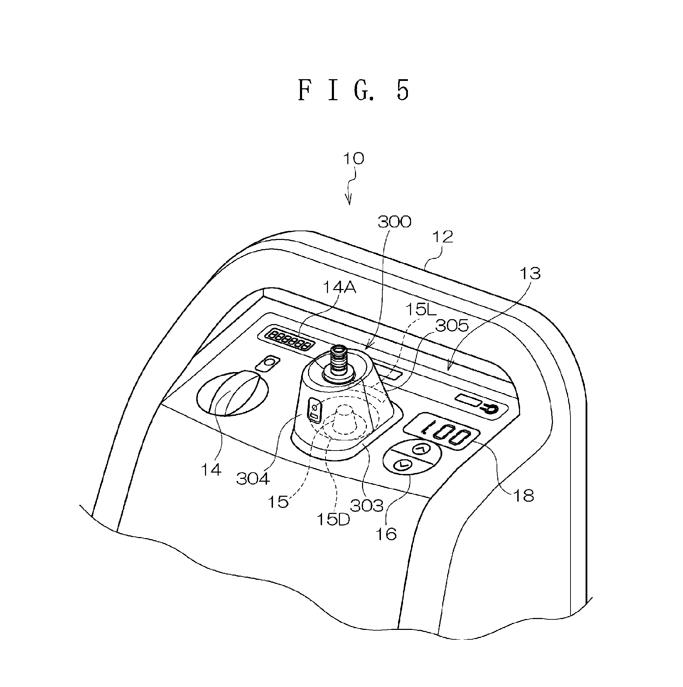

Next, the operation panel 13 of the oxygen concentrator 10 and the overheating detection unit 300 detachably attached to the operation panel 13 will be described with reference to FIGS. 5 and 6.
FIG. 5 is a perspective view showing the operation panel 13 of the oxygen concentrator 10 and the overheating detection unit 300. FIG. 6(A) is a perspective view showing the operation panel 13, and FIG. 6(B) is a plan view showing the operation panel 13.
Although the overheating detection unit 300 is not attached to the step portion 15D of the operation panel 13 shown in FIG. 2, FIGS. 5 and 6 show a state where the overheating detection unit 300 is attached to the step portion 15D of the operation panel 13.
As shown in FIGS. 5 and 6, the protection cover 303 which is a bottom portion of the overheating detection unit 300 is fitted into the step portion 15D. The protection cover 303 of the overheating detection unit 300 is held by being in close contact with the bottom portion 15F and the peripheral portion 15C of the step portion 15D shown in FIG. 2. The overheating detection unit 300 can be detachably connected to the oxygen outlet 15 and the coupler socket 23 so that the oxygen outlet 15 of the oxygen concentrator 10 communicates with the opening of the coupler socket 23 of the nasal cannula 22. Further, the overheating detection unit 300 has the role of reliably detecting a high-temperature environment when the user is inhaling oxygen using a cannula and is exposed to a fire or an abnormal high-temperature environment to thereby secure safety during use.

FIG. 7(A) is a perspective view as seen from the left front side of the overheating detection unit 300, FIG. 7(B) is a perspective view as seen from the right front side of the overheating detection unit 300, and FIG. 7(C) is an exploded perspective view of the overheating detection unit 300.
As shown in FIG. 7, the overheating detection unit 300 includes a housing 301, a main body 302 accommodated in the housing 301, and the protection cover 303 that covers the bottom portion of the main body 302. The housing 301 is held in the main body 302, and the protection cover 303 constitutes the bottom portion of the housing 301 and covers the main body 302.
Moreover, a front cover 304 as a first cover that constitutes the housing 301 and a rear cover 305 as a second cover are an outer housing that covers the side surfaces of the main body 302.
In the exploded perspective view of the overheating detection unit 300 of FIG. 7(C), the protection cover 303 is not illustrated.

The front cover 304 and the rear cover 305 of the housing 301 shown in FIG. 7(C) are formed of a plastic material (for example, a polycarbonate or an ABS which is a thermoplastic resin).
The front cover 304 includes a curved front surface 304A and a depressed upper surface 304B, and a bottom portion thereof is an opening 304C. The rear cover 305 includes a curved rear surface 305A and a depressed upper surface 305B, and a bottom portion thereof is an opening 305C. The front cover 304 and the rear cover 305 have such a tapered shape that the upper side is narrow and the lower side is wide, and the openings 304C and 305C are blocked by the protection cover 303.
The front cover 304 and the rear cover 305 has an inner space for covering the peripheral portion of the main body 302, and the upper surfaces 304B and 305B form a first opening 300H having an approximately circular shape. The oxygen outlet portion 310 of the main body 302 is exposed to the outside through the first opening 300H. The bottom portion of the main body 302 is exposed through the openings 304C and 305C. The oxygen outlet portion 310 having the function as the connecting portion has the same structure as the oxygen outlet 15 on the oxygen concentrator 10 shown in FIG. 2, having the function as the connecting portion, for example.

As shown in FIG. 7(A), a removal button 311 is disposed in the lower left portion of the front surface 304A of the front cover 304 and the lower left portion of the rear surface 305A of the rear cover 305.
As shown in FIG. 7(C), the main body 302 includes the oxygen outlet portion 310, a geared motor M, the removal button 311, a buzzer 313, a control board 314, a flexible resin tube (also called an internal tube) 315 formed of a polyvinyl chloride resin, having an outer diameter of 5 mm to 6 mm, an inner diameter of 4 mm to 6 mm, and a thickness of 0.8 mm to 1.2 mm, a cam 316, a battery 317, and the like. If the outer diameter of the tube 315 exceeds 6 mm, the blocking structure portion 400 becomes large and heavy, and as a result, the entire overheating detection unit 300 becomes large and heavy. If the outer diameter of the tube 315 is smaller than 5 mm, a flow resistance to the concentrated oxygen increases. Moreover, if the inner diameter of the tube 315 is smaller than 4 mm, the flow resistance to the concentrated oxygen increases. Further, if the thickness of the tube 315 exceeds 1.2 mm, driving force for driving the blocking structure portion 400 to block the tube 315 increases.
As shown in FIG. 7(C), the geared motor M has a speed reducer GM, and the speed reducer GM reduces the rotation speed of the geared motor M. The control board 314 is fixed by being hooked on a frame 318 of the main body 302. A test mode button 320, a green LED 321G, a yellow LED 321Y, a red LED 321R, and the like are mounted on the control board 314.

As shown in FIG. 7(C), the test mode button 320 is disposed so as to correspond to an elastic body 304E integrated with a recess 304F of the front cover 304 and a button operation portion 312B of a display 312 fitted into the recess 304F. The green LED, yellow LED 321Y, and red LED 321R are disposed so as to correspond to a LED display portion 312C of the display 312. In this manner, the user can press the test mode button 320 with the elastic body 304E interposed by pressing the button operation portion 312B with the finger. Moreover, the green, yellow, and red LEDs 321G, 321Y, and 321R can diffuse and emit green, yellow, and red beams through the LED display portion 312C of the display 312.
The display 312 is disposed on the front side of the front surface 304A of the front cover 304. In the display 312, since diffusion ink is applied to the LED display portion 312C, it is possible to diffuse light emitted from the LEDs in order to decrease directivity of light when the light emitting diodes (LEDs) on the main body 302 emit light through the opening 304C of the front cover 304. The display 312 can visually inform the user of various states by changing the emission colors of the three-color LEDs (the green, yellow, and red LEDs 321G, 321Y, and 321R), the states including a state where the overheating detection unit 300 operates normally (lighting or blinking of the green led 321G), an oxygen cut-off state when overheating is detected, and a battery replacement state. The buzzer 313 is a piezoelectric buzzer, for example.

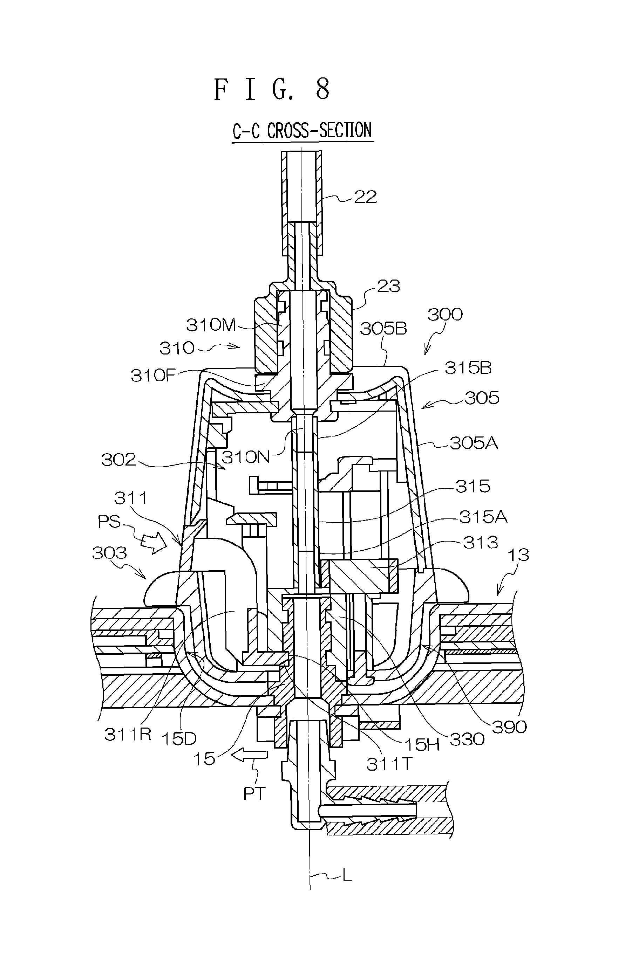

FIG. 8 shows a cross-section along line C-C, of the overheating detection unit 300 and the operation panel 13 shown in FIG. 6(B).
In FIG. 8, although, the step portion 15D (see FIG. 2) of the operation panel 13, the oxygen outlet 15 of the operation panel 13, the rear cover 305, the removal button 311, the tube 315, the oxygen outlet portion 310, and the like are shown, the geared motor M and the like are not shown. The oxygen outlet portion 310 is an oxygen outlet fitting formed of a metal, for example.
The protection cover 303 is held by being fitted into the step portion 15D of the operation panel 13, and the oxygen outlet 15 protrudes into the step portion 15D in a cylindrical form.
The oxygen outlet 15 is detachably connected to the connecting member 330 of the main body 302.
The connecting member 330 is detachably connected to one end 315A of the tube 315. The other end 315B of the tube 315 is detachably connected to an inner end 310N of the oxygen outlet portion 310. An outer end 310M of the oxygen outlet portion 310 is detachably connected to the coupler socket 23 of the nasal cannula 22 shown in FIG. 1. The oxygen outlet 15, the connecting member 330, the tube 315, and the oxygen outlet portion 310 are arranged along an axial direction L.

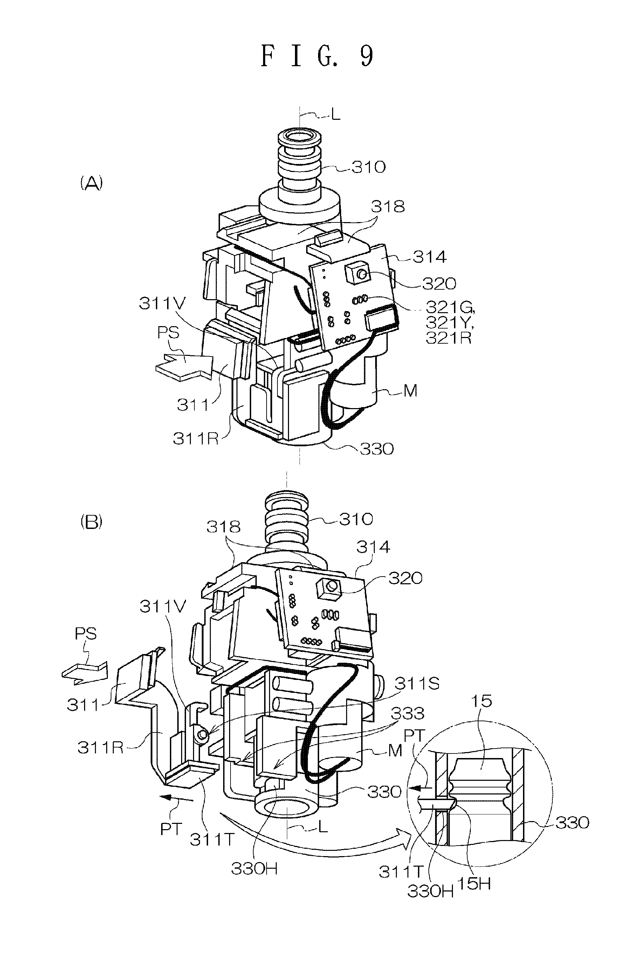

FIG. 9(A) is a perspective view showing the main body 302, and FIG. 9(B) is a perspective view showing the removal button 311 of the main body 302 in an exploded state.
As shown in FIGS. 8 and 9(B), the removal button 311 includes an L-shaped arm 311R, a claw portion 311T, and a boss portion 311S. The boss portion 311S is attached to the frame 333. In this sate, as shown in FIG. 8, the claw portion 311T passes through a hole 330H of the connecting member 330 and is fitted into a groove 15H of the oxygen outlet 15 with an appropriate load applied, whereby the overheating detection unit 300 is fixed to the oxygen outlet 15. Thus, the overheating detection unit 300 may not be removed in the state of being fitted into the step portion 15D.
When the overheating detection unit 300 is removed from the step portion 15D, the user presses the removal button 311 in the direction indicated by "PS" with the finger thereof, whereby a flexible portion 311V is bent about the boss portion 311S, and the claw portion 311T can be removed from the groove 15H of the oxygen outlet 15. Thus, the overheating detection unit 300 can be easily removed from the step portion 15D. In this manner, attachment and removal of the overheating detection unit 300 can be performed easily.

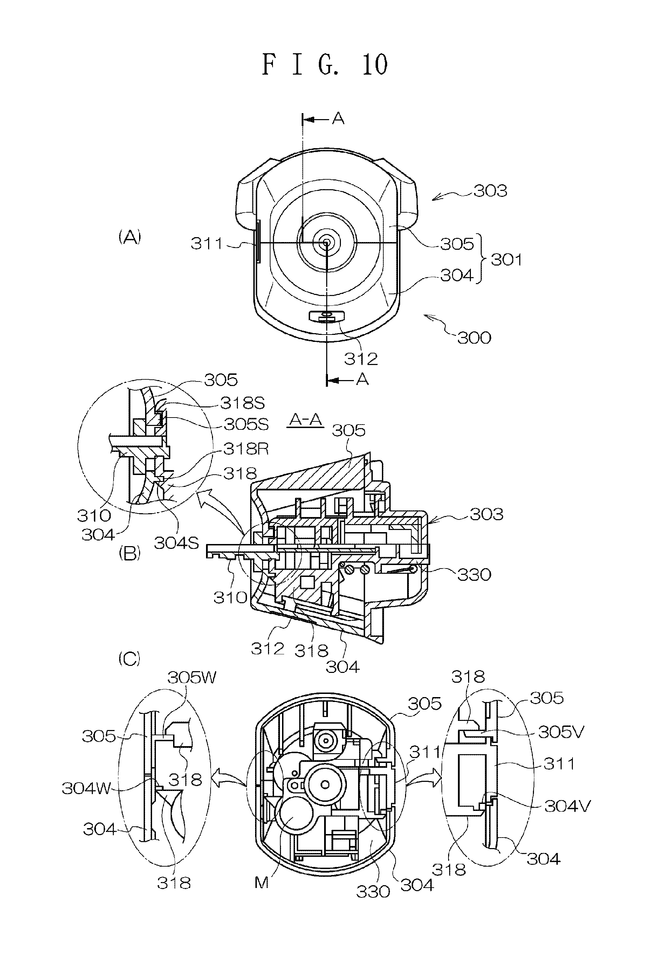

FIG. 10(A) is a plan view of the overheating detection unit 300, FIG. 10(B) is a cross-sectional view along line A-A, of the overheating detection unit 300 of FIG. 10(A), and FIG. 10(C) is a bottom view of the overheating detection unit 300 with the protection cover 303 excluded.
As shown in FIG. 10(B), the oxygen outlet portion 310 is fixed so as to be interposed between the front cover 304, the rear cover 305, and the frame 318. That is, the claw portion 304S of the front cover 304 and the claw portion 305S of the rear cover 305 are fitted into the recesses 318R and 318S of the frame 318, respectively, whereby the oxygen outlet portion 310 is interposed between the front cover 304, the rear cover 305, and the frame 318.

Moreover, as shown in FIG. 10(C), the front cover 304 and the rear cover 305 are fixed by the claw portions hooked on the frame 318, without using screws and bolts/nuts. That is, the front cover 304 has claw portions 304V and 304W, and the claw portions 304V and 304W are fixed by being fitted to the frame 318. Similarly, the rear cover 305 has claw portions 305V and 305W, and the claw portions 305V and 305W are fixed without the use of screws and bolts/nuts by being fitted to the frame 318. In this manner, the front cover 304 and the rear cover 305 are configured to be detachably attached using the frame 318 of the main body 302 so as not to be separated.

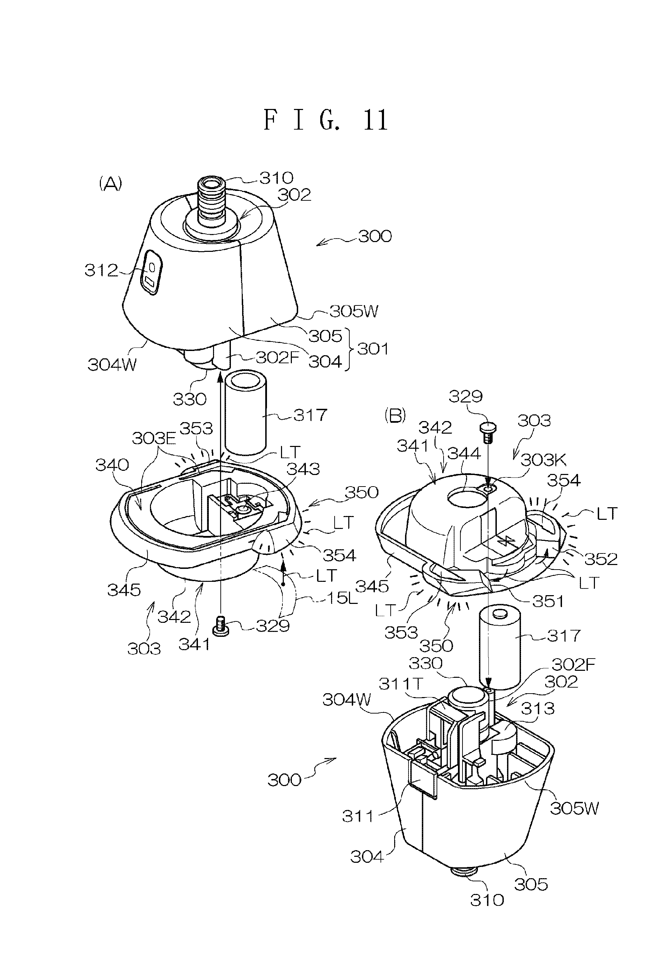

FIG. 11 is a perspective view showing the structure of the protection cover 303 and a replacement structure of the battery 317, in which FIG. 11 (A) shows an inner shape of the protection cover 303 and FIG. 11(B) shows an outer shape of the protection cover 303.
The protection cover 303 can be fixed to the main body 302 by inserting only one screw 329 into a female screw portion 302F of the main body 302 from a screw hole 303K of the protection cover 303.
As shown in FIG. 11(A), narrow grooves 303E are formed on an inner surface 340 of the protection cover 303, and a bottom side edge 304W of the front cover 304 and a bottom side edge 305W of the rear cover 305 are fitted to these narrow grooves 303E, whereby the protection cover 303, the front cover 304, and the rear cover 305 can be integrally assembled. In this manner, the protection cover 303 closes the bottom portions of the front cover 304 and the rear cover 305 and protects the main body 302.
When the user attaches a new battery 317 and replaces the battery 317, the user can easily perform a battery replacement operation just by removing one screw 329 to remove the protection cover 303, the front cover 304, and the rear cover 305. A lithium battery, for example, can be used as the battery 317.

Here, the structure of the protection cover 303 will be described with reference to FIG. 11.
As shown in FIG. 11, the protection cover 303 performs the role of covering and protecting the main body 302, the role of holding the overheating detection unit by being fitted to the step portion 15D of the operation panel 13 as shown in FIG. 8, and the role of a lens for guiding light.
The protection cover 303 is molded of a transparent resin (for example, an acryl resin, a polycarbonate resin, or the like). As shown in FIGS. 11(A) and 11(B), the protection cover 303 has the inner surface 340 and an outer surface 341. The narrow grooves 303E described above, a convex fitting portion 342, and an electric contact 343 are provided on the inner surface 340. A second circular opening 344 is formed in the convex fitting portion 342 as shown in FIG. 11(B), an end of the connecting member 330 is fitted to the second opening 344, and the end of the connecting member 330 is exposed to the outside of the protection cover 303 in order to be connected to the oxygen outlet 15.

As shown in FIGS. 11 (A) and 11(B), the protection cover 303 includes a peripheral portion 345 and a lens portion 350 for guiding lamp light. The peripheral portion 345 is formed in a portion that corresponds to a portion of the bottom side edge 304W of the front cover 304 and a portion of the bottom side edge 305W of the rear cover 305, and the lens portion 350 is formed in a portion that corresponds to a remaining portion of the bottom side edge 305W of the rear cover 305. The remaining portion of the bottom side edge 305W of the rear cover 305 is a portion that corresponds to an oxygen lamp 151 shown in FIG. 2.
The lens portion 350 shown in FIG. 11 includes a fist light introduction portion 351, a second light introduction portion 352, a first light guiding portion 353, and a second light guiding portion 354. When the protection cover 303 is held by being fitted to the step portion 15D of the operation panel 13 as shown in FIG. 6, the lens portion 350 is formed in an approximately fan shape so as to correspond to the oxygen lamp 15L shown in FIGS. 2 and 6.

As shown in FIG. 11 (A), light LT generated by the oxygen lamp 15L is introduced into the lens portion 350 from the first and second light introduction portions 351 and 352 shown in FIG. 11(B), and the light can be guided to the outside by the first and second light guiding portions 353 and 354. Thus, as shown in FIGS. 8(B) and 11 (A), in a state where the protection cover 303 is held by being fitted into the step portion 15D of the operation panel 13, the light LT generated by the oxygen lamp 15L can be easily observed with the naked eyes using the first and second light guiding portions 353 and 354. That is, since the light of the oxygen lamp 15L of the operation panel 13 of the oxygen concentrator 10 can be guided to a plurality of positions around the rear cover 305 which is the second cover, the user can easily observe the light of the oxygen lamp 15L with the naked eyes even when the user is located at approximately several meters from the oxygen concentrator 100.

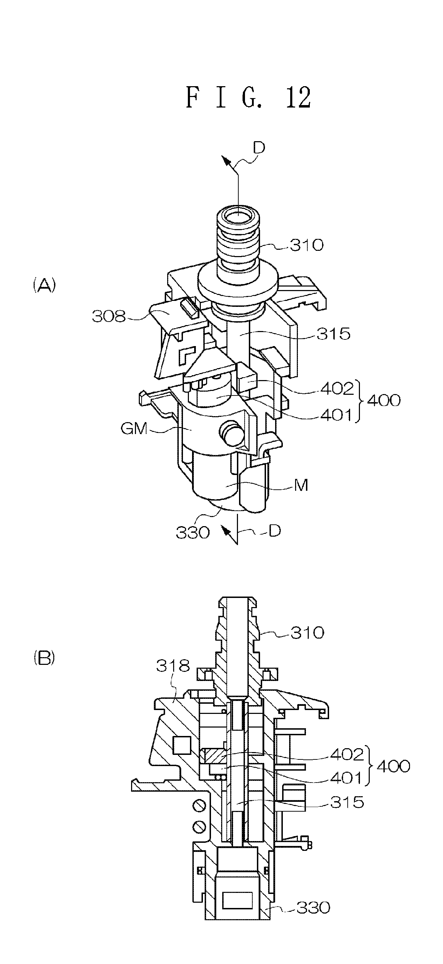

Next, the blocking structure portion 400 for blocking a halfway portion of the tube 315 shown in FIG. 8 will be described with reference to FIGS. 12 to 14.
FIG. 12(A) is a perspective view showing the main body 302 including the blocking structure portion 400, and FIG. 12(B) is a cross-sectional view along line D-D, of the main body 302.
The blocking structure portion 400 is used for pressing the tube 315 which is an example of a passage to block the tube 315. The blocking structure portion 400 includes a geared motor M as an electric motor which is an example of a driving portion, a cam 401 which is an example of an operating member, and a blocking member 402.
The geared motor M is a DC motor that includes a speed reducer GM, for example, and the rotation speed thereof is generally reduced to 60 rpm to 120 rpm, for example. The tube 315 is formed of a flexible and elastically deformable material (for example, a plastic material such as a polyvinyl chloride resin (PVC) or a silicon resin).
The cam 401 and the blocking member 402 are formed of a plastic material (for example, acrylonitrile butadiene styrene (ABS) or polyacetal (POM)). The cam 401 and the blocking member 402 are disposed near the tube 315, and the blocking member 402, the cam 401, and the geared motor M are arranged in parallel to the axial direction L in that order.

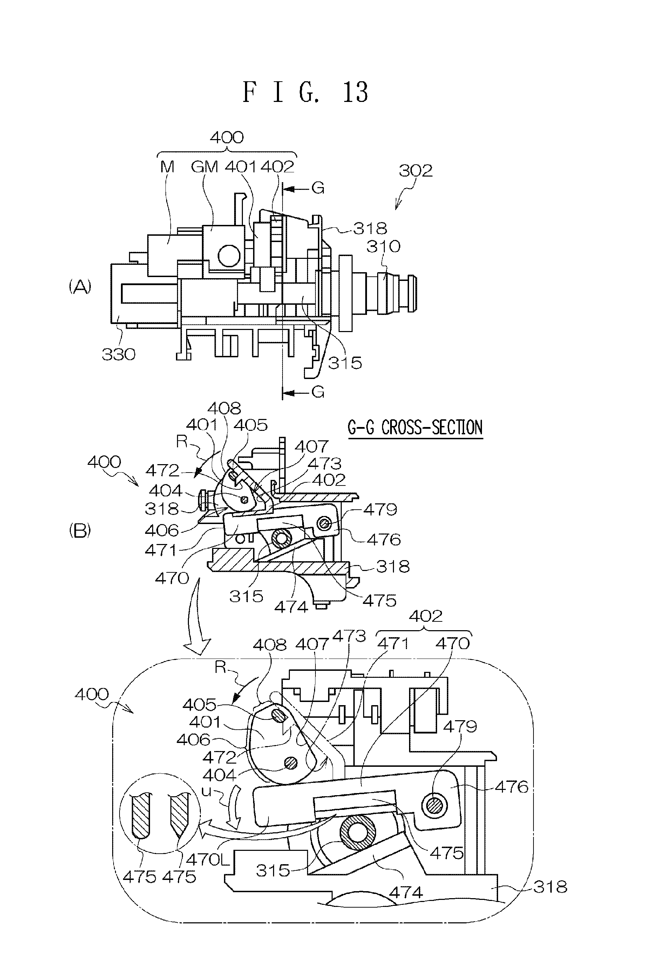

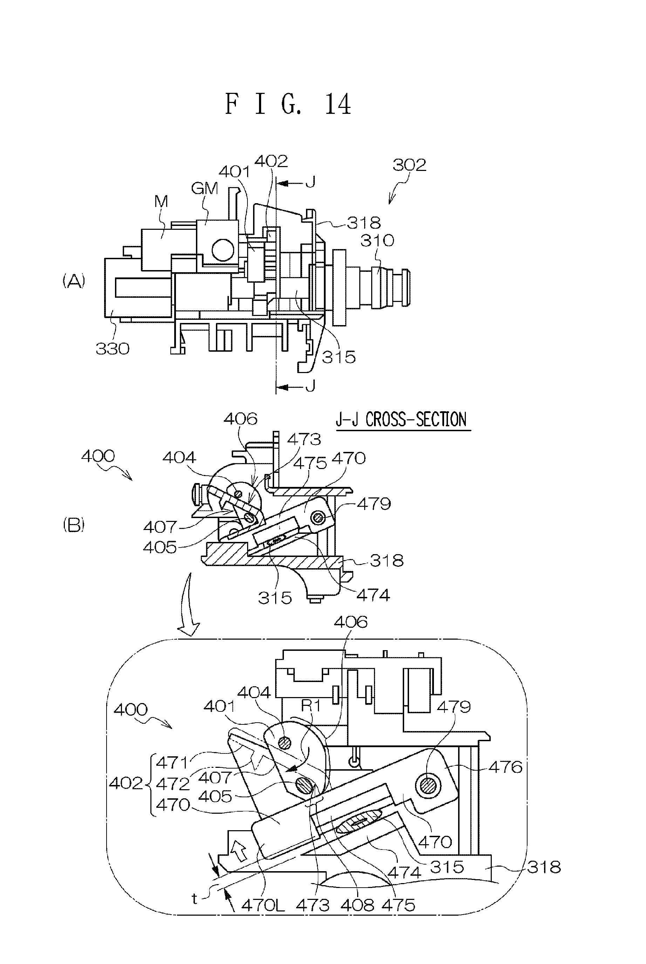

FIG. 13(A) shows a normal state where the tube 315 of the main body 302 is not pressed by the cam 401, that is, oxygen passes through the tube 315. FIG. 13 (B) is a cross-sectional view along line G-G, of the blocking structure portion 400 and the tube 315 of the main body 302.
Moreover, FIG. 14(A) shows a state where the tube 315 of the main body 302 is pressed and blocked by the cam 401, that is, abnormal overheating of the oxygen outlet portion 310 is detected so that oxygen cannot pass through the tube 315. FIG. 14(B) is a cross-sectional view along line J-J, of the blocking structure portion 400 and the tube 315 of the main body 302.

A preferred shape example of the cam 401 will be described with reference to FIGS. 13 and 14.
The cam 401 has a rotation shaft 404 at one end thereof. The rotation shaft 404 is configured to rotate in the direction indicated by R in relation to the frame 318. The rotation shaft 404 of the cam 401 is connected to an output shaft of the geared motor M. The cam 401 can rotate in the direction indicated by R as shown in FIG. 13(B) when the output shaft of the geared motor M rotates in a normal direction, and the cam 401 can rotate in the direction indicated by R as shown in FIG. 13(B) when the output shaft of the geared motor M rotates in a reverse direction.
A cam stopper pin 405 that restricts a rotation angle of the cam 401 is attached to the other end (rotating end) of the cam 401. Although the cam 401 has a curved cam following surface 406, since a convex cam following surface is not necessary on the opposite side of the cam following surface 406, a flat surface or a removed portion 407 that is substantially flat is formed on the opposite side.

In this manner, since the curved cam following surface 406 is required on only one side of the cam 401, and a curved cam following surface is not required on the opposite side of the cam following surface 406, the removed portion 407 is preferably secured. Thus, since the cam 401 having the removed portion 407 can decrease the size and weight of the cam 401 and can decrease the torque of the geared motor M for rotating the cam 401 as compared to a case where a convex curved cam following surface is formed on the entire periphery of the cam, it is possible to decrease the size of the geared motor M.
As shown in FIG. 13(B), another flat surface 408 is formed on a distal end of the cam 401. The reason why another flat surface 408 is formed in this manner is to maintain a state where the other flat surface 408 presses the blocking member 470 of the arm 402 as shown in FIG. 14(B) in a state where the cam 401 shown in FIG. 14(A) is rotated by 180° in the direction indicated by R. That is, since the other flat surface 408 has such a shape that the surface is substantially parallel to a blocking tool 470 of the arm 402, even if the supply of power from the battery 317 to the geared motor M is cut after the halfway portion of the tube 315 is blocked, the cam 401 will not rotated in the reverse direction indicated by R1. Thus, it is possible to reliably hold the blocked state of the tube 315 and to block oxygen in the tube 315.

Next, the shape of the blocking member 402 will be described with reference to FIGS. 13(B) and 14(B).
The blocking member 402 includes a cam rotation angle restricting portion 471 and the blocking tool 470. The cam rotation angle restricting portion 471 is adjacent to the cam 401, and a projection 472 protrudes from the inner surface of the cam rotation angle restricting portion 471. This projection 472 is a portion that receives the cam stopper pin 405 on the cam 401 in a tube open state shown in FIG. 13 (B) . In contrast, a portion 473 on the inner surface of the cam rotation angle restricting portion 471 is a portion that receives the cam stopper pin 405 in a tube blocked state shown in FIG. 14(B). In this manner, the cam 401 can rotate in the range of 180° corresponding to the angle between the tube open state shown in FIG. 13(B) and the tube blocked state shown in FIG. 14(B).

The blocking tool 470 is molded integrally with the cam rotation angle restricting portion 471, one end 470L of the blocking tool 470 is a movable end that moved downward in the direction indicated by U, and the other end 476 of the blocking tool 470 is attached to the central rotation shaft 479.
The blocking tool 470 has a pressing portion 475 for pressing against the tube 315, and the pressing portion 475 faces a fixing portion 474 of the frame 318. The tube 315 is disposed between the pressing portion 475 of the blocking tool 470 and the fixing portion 474. In this manner, when the blocking tool 470 is moved downward in the direction indicated by U from the tube open state shown in FIG. 13(B) to the tube blocked state shown in FIG. 14(B), the tube 315 is pressed to form a tube blocked state, and oxygen cannot pass through the tube 315. The gap t between the pressing portion 475 and the fixing portion 474 is 1.3 mm, for example.
As shown in FIG. 13(B), a distal end of the pressing portion 475 may have an acuate shape or a semi-circular shape, for example, so that the tube 315 can be easily crushed.

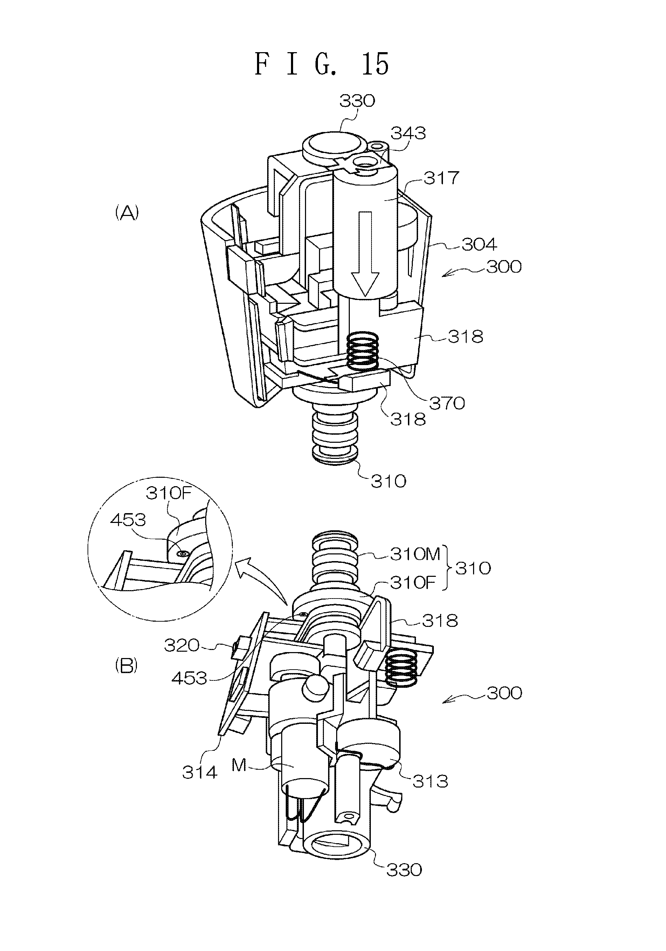

As shown in FIG. 15(A), the electric terminal 343 of the battery 317 is disposed inside the protection cover 303 as shown in FIG. 11(A). The other electric terminal 370 of the battery 317 is fixed to the frame 318.
As shown in FIG. 15(B), a thermistor 453 as a temperature sensor for detecting the temperature of the oxygen outlet portion 310 is disposed in a hole 310J at the bottom of a flange 310F of the oxygen outlet portion 310.

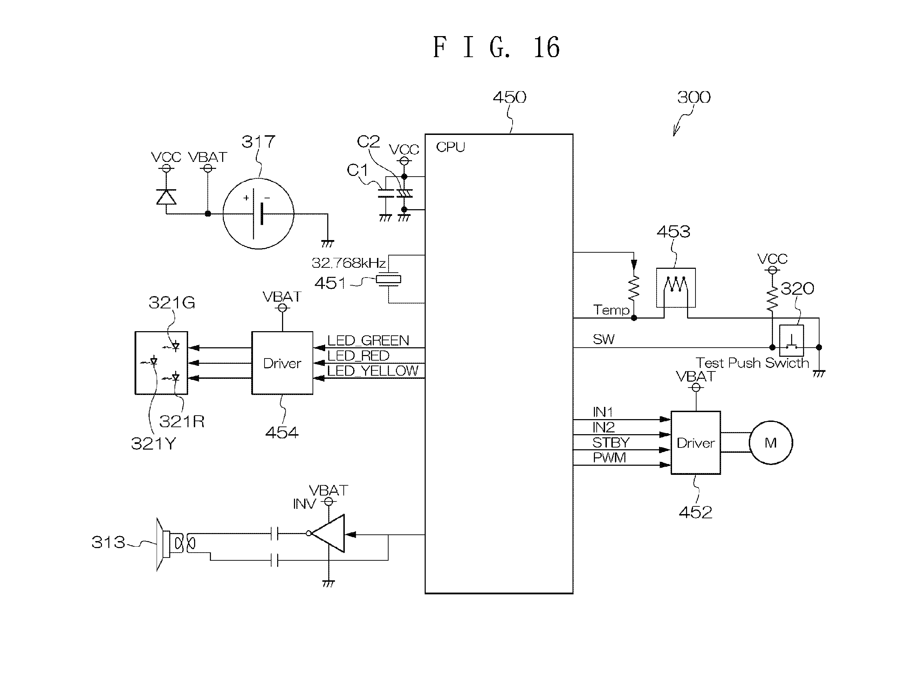

FIG. 16 shows an electric circuit of the overheating detection unit 300.
This electric circuit is mounted on the control board 314 shown in FIG. 7C, and a control unit (central processing unit (CPU)) 450 is electrically connected to capacitors C1 and C2, a reference frequency generator 451, the buzzer 313, a motor driver 452, the thermistor 453 as a temperature detection sensor, an LED driver 454, and the test mode switch 320. The power of the battery 317 is supplied to the motor driver 452, the LED driver 454, and the buzzer 313. The test mode switch 320 is also called a test switch and is pressed in order to test an overheating detection operation after the user attaches the overheating detection unit 300 to the operation panel 13 of the oxygen concentrator 10.
The thermistor 453 shown in FIGS. 15(B) and 16 detects the temperature of the overheating detection unit 300. The motor driver 452 shown in FIG. 16 drives the geared motor M so as to rotate in normal and reverse directions. The LED driver 454 shown in FIG. 16 drives the green, yellow, and red LEDs 321G, 321Y, and 321R.
The green LED 321G shown in FIG. 16 can inform the user of the fact that the overheating detection unit 300 operates normally by emitting green light through the display 312 shown in FIG. 11. The red LED 321R shown in FIG. 16 can inform the user of the oxygen cut-off state when overheating is detected by emitting red light through the display 312 shown in FIG. 11. The yellow LED 321Y shown in FIG. 16 can inform the user of the battery replacement state of the overheating detection unit 300 by emitting yellow light through the display 312 shown in FIG. 11. In this manner, it is possible to inform the user of different states by changing emission colors. As a result, the user can visually perceive respective states based on colors.
In this case, when the green, red, and yellow LEDs 321G, 321Y, and 321Y are lit, respective light beams are diffused from and displayed on the display 312 on the front side of the side surface 304A of the front cover 304 shown in FIG. 7 (A). That is, since diffusion ink is applied to the display 312, it is possible to diffuse the directivity of the light emitted from the light emitting diode (LED) and to diffuse the light emitted from the LED. In this manner, it is possible to obtain satisfactory visibility even when the LED is blinking.

Next, a use example of the overheating detection unit 300 described above will be described.

### <During Battery Attachment>

As shown in FIG. 11 (B), by removing only one screw 329 from the protection cover 303, the protection cover 303 can be easily removed from the front cover 304, the rear cover 305, and the main body 302. Moreover, as shown in FIG. 15(A), the battery 317 is attached to the main body 302. After that, when the protection cover 303 is fixed to the main body 302 using only one screw 329 shown in FIG. 11 (B), the bottom portions of the front cover 304 and the rear cover 305 are blocked, and the main body 302 can be covered by the front cover 304, the rear cover 305, and the protection cover 303. Thus, the overheating detection unit 300 is in an assembled state as shown in FIG. 7(A).
In this manner, since the protection cover 303 can be removed from and assembled into the front cover 304 and the rear cover 305 using only one screw 329, the user can easily perform a disassembling and assembling operation and the operation of attaching a new battery 317 and replacing an old battery 317 with a new battery 317.

When the battery 317 is attached to the main body 302 in the above described manner, the control unit 450 shown in FIG. 15 starts the system to first determine whether the battery 317 is consumed. In battery consumption determination, the motor driver 452 starts the geared motor M, and the measured voltage is lower than a predetermined voltage, the control unit 450 determines that the battery 317 is consumed, and the LED driver 454 shown in FIG. 16 causes the yellow LED 321Y to blink to visually urge the user to replace the battery.
In this case, when the yellow LED 321Y is lit, yellow light is diffused from and displayed on the display 312 on the front side of the side surface 304A of the front cover 304 shown in FIG 7(A). That is, since diffusion ink is applied to the display 312, it is possible to diffuse the directivity of the light emitted from the light emitting diode (LED) and to diffuse the light emitted from the LED. In this manner, it is possible to obtain satisfactory visibility even when the LED is blinking.

### <Overheating Detection Mode>

When a new battery is attached instead of a consumed battery, the control unit 450 of FIG. 16 executes "overheating detection mode". In an example of a normal operation in the "overheating detection mode", the control unit 450 checks the temperature of the oxygen outlet portion 310 using the thermistor 453 as a temperature sensor shown in FIG. 15(B) every predetermined second (for example, every 0.5 seconds) and checks the voltage of the battery 317 of FIG. 16 every 0.5 seconds. Further, the control 450 of FIG. 16 checks the number of operating days of the overheating detection unit 300 from the startup every 0.5 seconds and lights the green LED 321G for 25 mS every three seconds.
When the green LED 321G is lit, green light is diffused from and displayed on the display 312 on the front side of the side surface 304A of the front cover 304. That is, since diffusion ink is applied to the display 312, it is possible to diffuse the directivity of the light emitted from the light emitting diode (LED) and to diffuse the light emitted from the LED. In this manner, it is possible to obtain satisfactory visibility even when the LED is blinking. The control unit 450 repeatedly performs the above-described normal operation. In periods other than the normal operation in the "overheating detection mode", the control unit 450 is in a sleep state to prevent consumption of the battery 317, and overheating detection can be performed reliably.

Subsequently, as shown in FIG. 8, the protection cover 303 of the overheating detection unit 300 including the battery 317 is held by being fitted to the step portion 15D of the operation panel 13. In this case, the oxygen outlet 15 is detachably connected to the connecting member 330 of the main body 302. In this case, as shown in FIGS. 8 and 9, the claw portion 311T passes through the hole 330H of the connecting member 330 and is fitted into the groove 15H of the oxygen outlet 15 with an appropriate load applied, whereby the overheating detection unit 300 is fixed to the oxygen outlet 15. Thus, the overheating detection unit 300 may not be carelessly removed in the state of being fitted into the step portion 15D. In this manner, the protection cover 303 of the overheating detection unit 300 can be easily fixed to the step portion 15D by being fitted to the step portion 15D of the operation panel 13.
On the other hand, the outer end 310M of the oxygen outlet portion 310 is detachably connected to the coupler socket 23 of the nasal cannula 22 shown in FIG. 1.

### <During Overheating Detection>

When the overheating detection unit 300 is in the overheating detection mode described above, if the temperature of the oxygen outlet portion 310 detected by the thermistor 453 shown in FIG. 15(B) exceeds a predetermined temperature (for example, 40°C), the control unit 450 shown in FIG. 15 measures the temperature of the oxygen outlet portion 310 every 0.5 second. If the temperature of the oxygen outlet portion 310 exceeds a predetermined temperature (for example, 70°C), the user immediately presses a portion of the tube 315 shown in FIG. 13 (B) to FIG. 14 (B) to block the tube 314 to thereby cut the supply of oxygen in the tube 315.
Moreover, when the temperature of the oxygen outlet portion 310 is equal to or higher than a predetermined temperature (for example, 40°C) and the temperature rise rate exceeds a predetermined rise rate (for example, 1.0°C/second) for five seconds, the control unit 450 shown in FIG. 16 immediately blocks the tube 315 as shown in FIGS. 13 to 14 to cut oxygen. When the temperature of the oxygen outlet portion 310 is equal to or higher than a predetermined temperature (for example, 40°C) and the temperature rise rate is equal to or smaller than a predetermined rise rate (for example, 1.0°C/second), the control unit 450 shown in FIG. 15 proceeds to the above-described normal "overheating detection mode". In this manner, it is possible to prevent malfunctioning due to sunlight entering into the room in the summer or the like and heat radiation from heating appliances in the winter.

### <Oxygen Cut-off Operation>

When oxygen is cut during overheating detection, the tube 315 is put into a blocked state from the open state as shown in FIGS. 13 (B) and 14(B). That is, the projection 472 of the cam rotation restricting portion 471 receives the cam stopper pin 405 in the tube open state shown in FIG. 13(B). When the cam 401 is rotated about the rotation shaft 404 in the direction (counterclockwise direction) indicated by R, one end 470L of the blocking member 470 is moved downward in the direction indicated by U, and the pressing portion 475 of the blocking member 470 presses against the tube 315. Further, since the cam stopper pin 405 of the cam 401 abuts on a portion 473 on the inner surface of the cam rotation restricting portion 471, the cam 401 is prevented from further rotating in the direction indicated by R. In this manner, the tube 315 is blocked, whereby oxygen can be cut.

In the oxygen cut-off state shown in FIG. 14(B), since the flat surface 408 of the cam 401 is formed, the state where the flat surface 408 presses the blocking member 470 of the arm 402 is maintained. That is, the cam 401 has such a shape that the flat surface 408 is approximately parallel to the blocking member 470 of the arm 402. Thus, after the halfway portion of the tube 315 is blocked, even if the supply of power from the battery 317 to the geared motor M is cut so that the elastic returning force of the tube 315 is applied to the blocking member 470, the cam 401 will not be rotated in the reverse direction indicated by R1 by the elastic returning force. Thus, it is possible to reliably hold the blocked state of the tube 315 and to cut oxygen.
In this manner, the oxygen cut-off state can be maintained even when the remaining battery capacity is empty since the flat surface 408 is formed on the cam 401. Thus, it is possible to reliably maintain the oxygen cut-off state without ending the oxygen cut-off state and to prevent oxygen from leaking from the oxygen outlet portion 310 to thereby secure safety.

When oxygen is cut, the control unit 450 that controls the entire operation of the overheating detection unit 300 including a RAM, a flash ROM, and a microcomputer shown in FIG. 16 causes the red LED 321R to continuously emit red light to thereby visually inform the user of the fact that oxygen is cut. Further, the control unit 450 of FIG. 15 causes the buzzer 313 to output sound. However, the user can stop the operation of the buzzer 320 by pressing the test mode button 320 of FIG. 16.
In FIG. 14(B), when the control unit 450 of FIG. 16 rotates the geared motor M in the direction (clockwise direction) indicated by R1, since the state where the flat surface 408 of the cam 401 presses the blocking member 470 of the arm 402 can be released as shown in FIG. 13(B), the tube 315 can return to a state where it restores its shape due to the elastic returning force.

### <Battery Warning>

In this embodiment, an operation lamp, for example, is provided as an alarming portion so that the operation of the overheating detection unit can be checked when the voltage of the battery 317 shown in FIG. 16 decreases or the number of operating days of the overheating detection unit 300 exceeds one year. In this embodiment, the yellow LED 321Y which is the operation lamp blinks yellow light. However, once this battery warning is made, the battery warning is not cleared unless the battery 317 is removed from the main body 302. When a new battery 317 is attached instead of the old battery 317, the battery warning is reset, and the overheating detection unit returns to a normal state.

### <Anti-Sticking>

However, when the overheating detection unit 300 attached to the oxygen concentrator 10 does not operate the geared motor M for a long period of time, a phenomenon (so-called "anchoring phenomenon") in which the rotor of the geared motor M is stuck to the stator may occur. In order to prevent the phenomenon where the rotor is stuck to the stator, the output shaft of the geared motor M is rotated in the normal direction for 0.2 second, for example, the tube 315 is slightly crushed using the cam 401 as shown in FIG. 14(B), and then, the geared motor M is broken for 0.3 second (terminals are shorted). After that, the output shaft of the geared motor M is rotated in the reverse direction for 0.5 second so that the tube 315 is not blocked. This anti-sticking operation is executed every predetermined period (for example, every 30 days) by the microcomputer of the control unit 450. Moreover, the anti-sticking operation does not involve LED lighting and the operation of the buzzer 313 so that the user is not informed. In this manner, it is possible to prevent the phenomenon in which the rotor of the geared motor M is stuck to the stator to allow the geared motor M to operate reliably at any time. Thus, when the overheating detection unit 300 detects an overheating state, since the geared motor M can be reliably operated to cut oxygen, it is possible to secure operational reliability of the overheating detection unit 300.

### <Test Mode Button>

When the test mode button 320 shown in FIG. 16 is pressed during the normal operation in the overheating detection mode, the central control unit 450 performs the following control operation in order to reproduce the overheating detection state for a predetermined second (for example, for 15 seconds).
The output shaft of the geared motor M is rotated in the normal direction so that the cam 401 crushes the tube 315. The red LED 321R which is the operation lamp emits red light and the buzzer 313 outputs sound. The buzzer 313 output sound for the first five seconds only.
After the elapse of 15 seconds, the output shaft of the geared motor M is rotated in the reverse direction, the cam 401 is rotated to release blocking of the tube 315 as shown in FIG. 13(B), and oxygen supply through the tube 315 is resumed. The red LED 321R is unlit, the sound of the buzzer 313 is stopped, and the overheating detection unit 300 returns to its original normal operation.
Although the overheating detection unit 300 is configured to be attached to the step portion 15D of the operation panel 13 of the oxygen concentrator 10, since it is necessary to test and check the operation of the overheating detection unit 300 when attached, the test mode button 320 is prepared for the user to check the operation of the overheating detection unit 300. An electromagnetic induction coil may be provided to both the overheating detection unit 300 and the oxygen concentrator 100 instead of the battery 317 so as to supply power from the oxygen concentrator 100 to the overheating detection unit 300.

The present invention is not limited to the respective embodiments described above, but can be applied to a membrane separation-type oxygen concentrator that uses a membrane that selectively permeates oxygen. Moreover, the overheating detection unit can be applied to an oxygen supply device that uses an oxygen cylinder.
Part of constituent components described in the above embodiments may be omitted, and combinations with other components that are not described above also fall within the scope of the present invention.

### Reference Signs List

- 10:: OXYGEN CONCENTRATOR
- 15:: OXYGEN OUTLET
- 300:: OVERHEATING DETECTION UNIT
- 301:: HOUSING
- 302:: MAIN BODY
- 303:: PROTECTION COVER
- 304:: FRONT COVER
- 305:: REAR COVER
- 310:: OXYGEN OUTLET PORTION
- 315:: TUBE (EXAMPLE OF PASSAGE)
- 330:: CONNECTING MEMBER
- 400:: BLOCKING STRUCTURE PORTION
- 401:: CAM (EXAMPLE OF OPERATING MEMBER)
- 402:: BLOCKING MEMBER
- 406:: CAM FOLLOWING SURFACE
- 407:: REMOVED PORTION OF CAM
- 408:: FLAT SURFACE OF CAM
- 453:: TEMPERATURE SENSOR
- M:: GEARED MOTOR (EXAMPLE OF DRIVING PORTION)

## Claims

1. An overheating detection unit disposed between an oxygen outlet of an oxygen concentrator that generates concentrated oxygen from raw material air and that has the oxygen outlet outputting the concentrated oxygen and a nasal cannula having a coupler socket, or between the oxygen outlet and the coupler socket provided at one end of a tube that is connected to the nasal cannula, so as to detect an overheating state,
the overheating detection unit comprising:
a main body including a connecting member connected to the oxygen outlet and also including an oxygen outlet portion connected to the coupler socket; and
a housing covering the main body,
the main body including:
a temperature sensor disposed in the oxygen outlet portion so as to detect a temperature of the oxygen outlet portion; and
a blocking structure portion blocking a passage between the connecting member and the oxygen outlet portion so as to cut supply of oxygen when the temperature sensor detects a temperature that is equal to or higher than a predetermined temperature.

2. The overheating detection unit according to claim 1, wherein
the housing includes a first cover that is fixed to the main body, a second cover that is fixed to the main body and detachably attached to the first cover, and a protection cover that is fixed to the main body so as to cover bottom portions of the first and second covers, and
a first opening for exposing the oxygen outlet portion is formed in the first and second covers, and a second opening for exposing an end of the connecting member is formed in a bottom portion of the protection cover.

3. The overheating detection unit according to claim 2, wherein the protection cover is detachably fixed to the main body by one screw.

4. The overheating detection unit according to claim 2 or 3, wherein
the protection cover has a convex fitting portion that is fitted to a step portion formed in the oxygen concentrator in a state where the oxygen outlet is connected to the connecting member.

5. The overheating detection unit according to any of claims 2 to 4, wherein
the protection cover is formed of a transparent resin, and the protection cover has a lens portion for guiding light of an oxygen lamp provided in the oxygen concentrator so as to emit light to display that oxygen is output from the oxygen outlet to a periphery of the overheating detection unit.

6. The overheating detection unit according to claim 5, wherein the lens portion guides the light of the oxygen lamp of the oxygen concentrator to a plurality of positions of an outer circumference of the second cover.

7. The overheating detection unit according to any of claims 1 to 6, wherein
the blocking structure portion includes a blocking member that presses an elastically deformable tube that connects the connecting member and the oxygen outlet portion to thereby cut the supply of oxygen.

8. The overheating detection unit according to any of claims 1 to 7, further comprising:
a display that is provided on the housing so as to inform, by changing emission colors, a user of a normal operation state, a cut-off state of the oxygen when overheating is detected, and a battery replacement state.

9. An oxygen concentrator comprising:
a compressed air generator compressing raw material air and generating compressed air;
an oxygen outlet outputting oxygen obtained from the compressed air; and
the overheating detection unit according to any of claims 1 to 8.

10. An overheating detection unit that is disposed between an oxygen outlet of an oxygen cylinder and a nasal cannula having a coupler socket, or between the oxygen outlet and the coupler socket provided at one end of a tube that is connected to the nasal cannula, so as to detect an overheating state,
the overheating detection unit comprising:
a main body including a connecting member connected to the oxygen outlet and an oxygen outlet portion connected to the coupler socket; and
a housing that covers the main body,
the main body including:
a temperature sensor disposed in the oxygen outlet portion so as to detect a temperature of the oxygen outlet portion; and
a blocking structure portion that blocks a passage between the connecting member and the oxygen outlet portion so as to cut supply of oxygen when the temperature sensor detects a temperature that is equal to or higher than a predetermined temperature.
